# EUROPEAN PATENT APPLICATION

(11) **EP 0 728 724 A1**
(43) Date of publication of application: **28.08.1996**
(21) Application number: 96102586.3
(22) Date of filing: 21.02.1996
(51) Int. Cl.: C07C 43/168, C07C 43/162, C07C 41/16, C08F 4/649

(54) **Diethers suitable for use in the preparation of Ziegler-Natta catalysts**

(30) Priority: 21.02.1995 IT MI950316
(71) Applicant: MONTELL NORTH AMERICA INC., New Castle County Delaware (US)
(72) Inventor: Morini, Giampiero, I-27058 Voghera, Pavia (IT); Cristofori, Antonio, I-45030 Occhiobello, Rovigo (IT)
(74) Representative: Zumstein, Fritz, Dr.

(57) **Abstract**

Diethers suitable for use in the preparation of Ziegler-Natta catalysts having the formula: 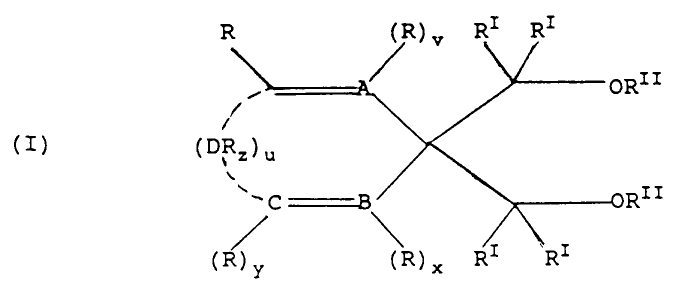 where A, B, C, and D are carbon atoms or heteroatoms, v, x, and y are 0 or 1; u and z are either 0, 1, or 2; the radicals R and R^{I}, equal or different, are H; halogens; linear or branched alkyl radicals; cycloalkyl, aryl, alkylaryl, and aralkyl radicals; the R^{II} radicals, equal or different, are linear or branched alkyl radicals; cycloalkyl, aryl, alkylaryl, and aralkyl radicals; at least two R radicals can be bonded to one another to form saturated or unsaturated condensed cyclic structures, optionally substituted with R^{III} radical selected from halogens; linear or branched alkyl radicals; cycloalkyl, aryl, alkylaryl, and aralkyl radicals; said radicals from R to R^{III} optionally containing at least one heteroatom.

## Description

The present invention concerns a new class of diethers and a new process for their synthesis.

The diethers of the present invention are particularly suited for the preparation of Ziegler-Natta catalysts to which they confer qualities far superior, in terms of balanced activity and stereospecificity in the (co)polymerization of olefins, to those that are obtained with the ethers known in the art. By olefins one refers in particular to the CH₂=CHR compounds where R is hydrogen, or a C₁-C₆ alkyl or aryl radical.

The diethers of the present invention have the following general formula (I) 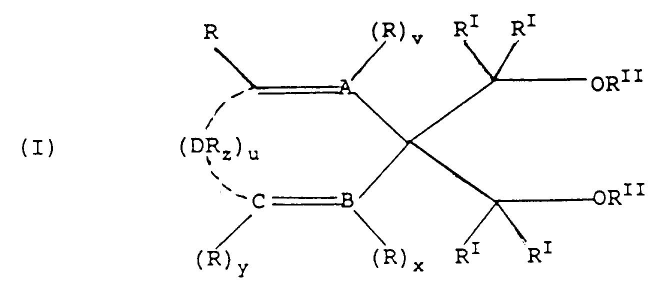 where A, B, C, and D are carbon atoms or heteroatoms selected from the group consisting of N, O, Si and S; v, x, and y are 0 or 1; u and z are either 0, 1, or 2;
provided that when u = 0:
i) A, B, and C are carbon atoms and v, x, and y are equal to 1; or
ii) A is a nitrogen atom, B and C are carbon atoms, v is equal to 0, and x and y are equal to 1; or
iii) A and B are nitrogen atoms, C is a carbon atom, v and x are equal to 0, and y is equal to 1; or
iv) A and B are carbon atoms, C is a nitrogen atom, v and x are equal to 1, and y is equal to 0;
when u = 1:
1) A, B, C, and D are carbon atoms, v, x, and y are equal to 1, and z is equal to 2; or
2) A and B are carbon atoms, C is a nitrogen atom, D is an oxygen atom, v and x are equal to 1, y and z are equal to 0; or
3) A, B, and C are carbon atoms, D is an oxygen, nitrogen, sulfur, or silicon atom, v, x, and y are equal to 1, and z is equal to 0 when D is an oxygen or sulfur atom, equal to 1 when D is a nitrogen atom, and equal to 2 when D is a silicon atom;
when u = 2:
A, B, and C are carbon atoms, D represents two carbon atoms bonded to each other by a single or double bond, v, x, and y are equal to 1, and z is 1 when the pair of carbon atoms D is bonded by a double bond, and z is 2 when said pair is bonded by a single bond;
radicals R and R^{I}, equal or different, are hydrogen; halogens, preferably Cl and F; C₁-C₂₀ alkyl radicals, linear or branched; C₃-C₂₀ cycloalkyl, C₆-C₂₀ aryl, C₇-C₂₀ alkylaryl and C₇-C₂₀ aralkyl radicals; the R^{II} radicals, equal or different, are C₁-C₂₀ alkyl radicals, linear or branched; C₃-C₂₀ cycloalkyl, C₆-C₂₀ aryl, C₇-C₂₀ alkylaryl and C₇-C₂₀ aralkyl radicals, and two or more of the R radicals can be bonded to each other to form condensed cyclic structures, saturated or unsaturated, optionally substituted with R^{III} radicals wherein R^{III} is a halogen, preferably Cl and F; C₁-C₂₀ alkyl radicals, linear or branched; C₃-C₂₀ cycloalkyl, C₆-C₂₀ aryl, C₇-C₂₀ alkylaryl and C₇- C₂₀ aralkyl radicals; said radicals from R to R^{III} optionally containing one or more heteroatoms as substitutes for carbon or hydrogen atoms, or both.

The heteroatoms optionally present in the radicals from R to R^{III} are preferably selected from the group consisting of N, O, S, P, Si, and halogens, in particular Cl and F.

A preferred group of the compounds of general formula (I) consists of the compounds of general formula: 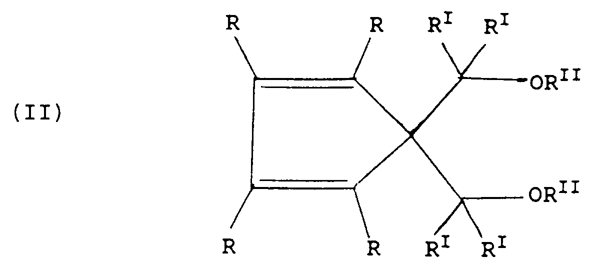 where the radicals from R to R^{II} are as defined above for formula (I).

In particular, two or more of the R radicals can be bonded to each other to form one or more condensed cyclic structures, preferably benzenic, optionally substituted by R^{III} radicals having the meaning specified above for formula (I).

Specially preferred are the compounds of formula: 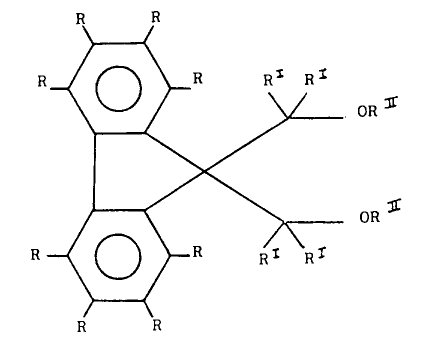 where the R radicals, equal or different, are hydrogen; halogens, preferably Cl and F; C₁-C₂₀ alkyl radicals, linear or branched; C₃-C₂₀ cycloalkyl, C₆-C₂₀ aryl, C₇-C₂₀ alkylaryl and C₇-C₂₀ aralkyl radicals, optionally containing one or more heteroatoms selected from the group consisting of N, O, S, P, Si and halogens, in particular Cl and F, as substitutes for carbon or hydrogen atoms, or both; the radicals R^{I} and R^{II} are as defined above for formula (I).

Specific examples of compounds comprised in formula (II) are:
1,1-bis(methoxymethyl)-cyclopentadiene;
1,1-bis(methoxymethyl)-2,3,4,5-tetramethylcyclopentadiene;
1,1-bis(methoxymethyl)-2,3,4,5-tetraphenylcyclopentadiene;
1,1-bis(methoxymethyl)-2,3,4,5-tetrafluorocyclopentadiene;
1,1-bis(methoxymethyl)-3,4-dicyclopentylcyclopentadiene;
1,1-bis(methoxymethyl)indene;
1,1-bis(methoxymethyl)-2,3-dimethylindene;
1,1-bis(methoxymethyl)-4,5,6,7-tetrahydroindene;
1,1-bis(methoxymethyl)-2,3,6,7-tetrafluoroindene;
1,1-bis(methoxymethyl)-4,7-dimethylindene;
1,1-bis(methoxymethyl)-3,6-dimethylindene;
1,1-bis(methoxymethyl)-4-phenylindene;
1,1-bis(methoxymethyl)-4-phenyl-2-methylindene;
1,1-bis(methoxymethyl)-4-cyclohexylindene;
1,1-bis(methoxymethyl)-7-(3,3,3-trifluoropropyl)indene;
1,1-bis(methoxymethyl)-7-trimethylsilylindene;
1,1-bis(methoxymethyl)-7-trifluoromethylindene;
1,1-bis(methoxymethyl)-4,7-dimethyl-4,5,6,7-tetrahydroindene;
1,1-bis(methoxymethyl)-7-methylindene;
1,1-bis(methoxymethyl)-7-cyclopenthylindene;
1,1-bis(methoxymethyl)-7-isopropylindene;
1,1-bis(methoxymethyl)-7-cyclohexylindene;
1,1-bis(methoxymethyl)-7-tert-butylindene;
1,1-bis(methoxymethyl)-7-tert-butyl-2-methylindene;
1,1-bis(methoxymethyl)-7-phenylindene;
1,1-bis(methoxymethyl)-2-phenylindene;
1,1-bis(methoxymethyl)-1H-benz[e]indene;
1,1-bis(methoxymethyl)-1H-2-methylbenz[e]indene;
9,9-bis(methoxymethyl)fluorene;
9,9-bis(methoxymethyl)-2,3,6,7-tetramethylfluorene;
9,9-bis(methoxymethyl)-2,3,4,5,6,7-hexafluorofluorene;
9,9-bis(methoxymethyl)-2,3-benzofluorene;
9,9-bis(methoxymethyl)-2,3,6,7-dibenzofluorene;
9,9-bis(methoxymethyl)-2,7-diisopropylfluorene;
9,9-bis(methoxymethyl)-1,8-dichlorofluorene;
9,9-bis(methoxymethyl)-2,7-dicyclopentylfluorene;
9,9-bis(methoxymethyl)-1,8-difluorofluorene;
9,9-bis(methoxymethyl)-1,2,3,4-tetrahydrofluorene;
9,9-bis(methoxymethyl)-1,2,3,4,5,6,7,8-octahydrofluorene;
9,9-bis(methoxymethyl)-4-tert-butylfluorene;
1,1-bis(1'-butoxyethyl)-cyclopentadiene;
1,1-bis(1'-isopropoxy-propyl)cyclopentadiene;
1-methoxymethyl-1-(1'-methoxyethyl)-2,3,4,5-tetramethylcyclopentadiene;
1,1-bis(α-methoxybenzyl)indene;
1,1-bis(phenoxymethyl)indene;
1,1-bis(1'-methoxyethyl)-5,6-dichloroindene;
1,1-bis(phenoxymethyl)-3,6-dicyclohexylindene;
1-methoxymethyl-1-(1'-methoxyethyl)-7-tert-butylindene;
1,1-bis[2-(2'methoxypropyl)]-2-methylindene;
3,3-bis(methoxymethyl)-3H-2-methylbenz[e]indene;
9,9-bis(α-methoxybenzyl)fluorene;
9,9-bis(1'-isopropoxy-butyl)-4,5-diphenylfluorene;
9,9-bis(1'-methoxyethyl)fluorene;
9-(methoxymethyl)-9-(1-methoxyethyl)-2,3,6,7-tetrafluorofluorene;
9-methoxymethyl-9-pentoxymethylfluorene;
9-methoxymethyl-9-ethoxymethylfluorene;
9-methoxymethyl-9-(1'-methoxyethyl)-fluorene;
9-methoxymethyl-9-[2-(2-methoxypropyl)]-fluorene;
5,5-bis(methoxymethyl)-1,5-pyrindine; and
5,5-bis(methoxymethyl)-6,7-dimethyl-1,5-pyrindine.

Other examples of 1,3-diethers comprised in formula (I) are:
1,1-bis(methoxymethyl)-2,5-cyclohexadiene;
1,1-bis(methoxymethyl)-benzonaphthene;
4,4-bis(methoxymethyl)-4H-cyclopenta[d,e,f]phenanthrene;
9,9-bis(methoxymethyl)-9-10-dihydroanthracene;
7,7-bis(methoxymethyl)-7H-benz[d,e]anthracene;
4,4-bis(methoxymethyl)-1-phenyl-1,4-dihydronaphthalene
5,5-bis(methoxymethyl)-1,3,6-cycloheptatriene;
5,5-bis(methoxymethyl)-10,11-dihydro-5H-dibenzo[a,d] cycloheptene;
5,5-bis(methoxymethyl)-5H-dibenzo[a,d]cycloheptene;
9,9-bis(methoxymethyl)xanthene;
9,9-bis(methoxymethyl)-2,3,6,7-tetramethylxanthene;
9,9-bis(methoxyisobutyl)thioxanthene;
4,4-bis(methoxymethyl)-1,4-pyran;
9,9-bis(methoxymethyl)-N-tert-butyl-9,10-dihydroacridine;
4,4-bis(methoxymethyl)-1,4-chromene;
4,4-bis(methoxymethyl)-1,2,4-oxazine;
1,1-bis-(methoxymethyl)benzo-2,3,1-oxazine;
2.2-bis(methoxymethyl)-3,4,5-trifluoroisopyrrole; and
4,4-bis(1'-methoxyethyl)benzo-N-phenyl-1,4-dihydropyridene.

The diethers of the present invention can be synthesized by various reactions. One of them consists of causing to react, in the presence of a base, a compound of the following general formula: 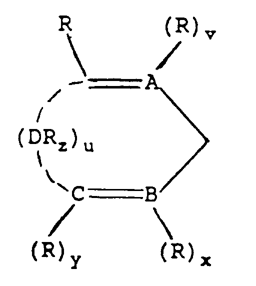 where u, v, x, y, z, A, B, C, D, and the R radicals are as defined for general formula (I), with compounds of the formula XC(R^{I})₂-OR^{II}, where X represents Cl, Br, and I, and R^{I} and R^{II} are as defined for general formula (I). However, this way of synthesizing has the inconvenience of producing low yields.

The diethers of the present invention can also be synthesized from the corresponding diols by an etherification reaction as described, for example, in European patent applications EP-A-361493 and EP-A-487035, both filed on behalf of the Himont Inc. The etherification reaction described in these applications requires that a diol, or the corresponding alkaline alcoholate, in a suitable organic solvent is reacted with an R^{II}X compound, or a (R^{II})₂SO₄ compound, where R^{II} is as defined for general formula (I), and X is Cl, Br, I, CH₃SO₃, C₆H₅-SO₃, or p-CH₃-C₆H₄-SO₃, in the presence of a base. According to the examples of said applications the etherification reaction is carried out by mixing the diol, or corresponding alkaline alcoholate, with the base in an organic solvent, and then adding the R^{II}X or (R^{II})₂SO₄ compound. Optionally base and R^{II}X or (R^{II})₂SO₄ compound can added subsequently.

This reaction produces yields that do not exceed 80%, and in same cases requires long synthesis periods.

Now has been found a synthesis process that allows one to obtain the diethers of the present invention with higher etherification yields. Also, less reaction time is needed for the etherification reaction. Said process can be used for the synthesis of the propane-1,3-diethers described in the above mentioned European patent application EP-A-361493.

Another object of the present invention is the process for the synthesis of diethers of general formula (III) 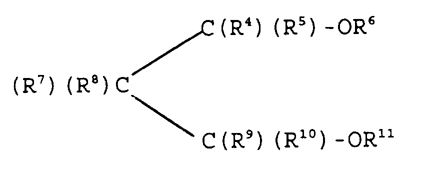 where the R⁷ and R⁸ radicals, equal or different, are hydrogen or C₁-C₂₀ alkyl radicals, linear or branched; C₃-C₂₀ cycloalkyl, C₆-C₂₀ aryl, C₇-C₂₀ alkylaryl and C₇-C₂₀ aralkyl radicals; the R⁴, R⁵, R⁹, and R¹⁰ radicals, equal or different, have the same meaning as defined for the R⁷ and R⁸ radicals; the R⁶ and R¹¹ radicals have the same meaning as the R⁷ and R⁸ radicals except for the hydrogen; moreover, two or more radicals from R⁴ to R¹⁰ can be bonded to form a cyclic structure; said radicals from R⁴ to R¹¹ optionally containing one or more heteroatoms, as substitutes for one or more carbon or hydrogen atoms, or both, selected from N, O, S, P, Si and halogens, preferably Cl and F.

Said process comprises the following steps:
a) mixing a diol of general formula (IV) 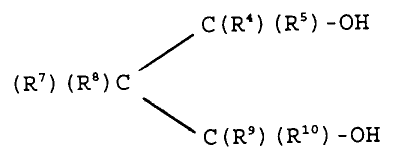 where the radicals from R⁴ to R¹⁰ are as defined for general formula (III), with a compound or mixture of compounds selected from the compounds of general formulae R⁶X (V), or general formula R¹¹X (VI), where X is Cl, Br, I, CH₃SO₃, C₆H₅-SO₃, or p-CH₃-C₆H₄-SO₃, or of general formula (R⁶)₂SOₙ (VII), or of general formula (R¹¹)₂SOₙ (VIII), where R⁶ and R¹¹ have the meaning as defined for general formula (III), and n is 3 or 4, in a solvent which is basically nonreactive toward the reagents; and then
b) adding a base which is substantially inert towards the compounds of general formulae (V) to (VIII), and is capable of forming the alcoholated derivative of the corresponding diol (IV) under the reaction conditions.

Preferably, the above mentioned bases are selected from the bases of alkaline metals. Examples of bases that can be used in the process of the present invention are sodium hydride and sodium hydroxide. In the specific case where sodium hydroxide is used, the preferred solvent is the dimethyl sulfoxide.

Preferably the base is added gradually. For example one can introduce the base in a period of time ranging from 10 minutes to 4 hours.

Examples of solvents suitable for use in the process of the present invention include tetrahydrofuran, dimethyl sulfoxide, diethyl ether, aliphatic hydrocarbons, such as pentane, heptane, hexane, or aromatic hydrocarbons such as toluene and benzene, and dimethylformamide.

Specific reaction conditions, such as temperature and pressure, are not crucial for the reaction to occur; for example the temperature can range from 0° to 100°C, and the operation can take place at ambient pressure.

It is preferable to carry out the above mentioned reaction by using quantities of the compound or mixture of compounds of formulae (V) to (VIII) in excess with respect to the diol (IV). In particular, the molar ratio of the diol to the compound or mixture of compounds (V)-(VIII) is preferably comprised from 1:3 to 1:15.

Examples of synthesis of diols of formula (IV) are already known in literature; for example synthesis processes are disclosed in above cited European patent applications EP-A-361493 and EP-A-487035, both filed on behalf of Himont Inc.

The diols corresponding to the diethers of formula (I) can be prepared according to various known synthesis processes, for example, by aldol condensation of the corresponding unsaturated cyclic compounds, such as fluorene, indene, cyclopentadiene, with proper aldehydes (see Acta Chemica Scandinavica 21, 1967, pp. 718-720, for example).

Examples of specific diols corresponding to the diethers of formula (I) and their related synthesis disclosed in literature, are 9,9-bis(hydroxymethyl)fluorene (see Acta Chemica Scandinavica 21, 1967, pp. 718-720), 9-hydroxymethyl-9-(α-nethyl)hydroxymethylfluorene (see Chemical Abstract, CAS number: 101168-93-8), 9-(α-methyl)hydroxymethyl-9-(α'-methyl)hydroxymethylfluorene (see Beilstein, registration number: 101594-61-0), and 9-(α-phenyl)hydroxymethyl-9-(α'-phenyl)hydroxymethylfluorene (see Beilstein, registration number: 103210-68-0).

The following examples are given in order to illustrate and not limit the present invention.

### Synthesis of 9,9-bis(hydroxymethyl)fluorene

Into a 500 mL flask, in anhydrous atmosphere, are introduced in order: 100 mL of dimethyl sulfoxide (DMSO) distilled on CaH, 8 g of paraformaldehyde (rendered anhydrous at ambient temperature and at a pressure of 2 torr for 8 hours), and 1.4 g of sodium ethylate dissolved in 6 mL of ethanol.

After having cooled the suspension by placing the flask in an ice bath (the melt temperature of the DMSO/EtOH mixture is 13°C) and while maintaining the suspension under agitation, are added threto over a period of thirty seconds, 100 mL of a solution of 16 g of fluorene in DMSO.

After 3 minutes from the beginning of the addition of the solution of fluorene in DMSO, the reaction is stopped by adding 1.5 mL of 37% aqueous HCl, and then the resulting mixture is diluted with 400 mL of water.

The mixture is saturated with NaCl, and 9,9-bis(hydroxymethyl)fluorene is extracted therefrom with ethyl acetate. The organic phase is then rendered anhydrous with anhydrous Na₂SO₄ and the solvent is flashed off. After toluene crystallization, 15.2 g of product (yield: 70%) is obtained.

The ¹H-NMR spectrum of the product in CDCl₃, at 200 MHz and with tetramethylsilane (TMS) as internal standard, shows the following:

| | | |
|---|---|---|
| 7.77 ppm, | doublet, | 2H aromatics |
| 7.62 ppm, | doublet, | 2H aromatics |
| 7.41 ppm, | triplet, | 2H aromatics |
| 7.32 ppm, | triplet, | 2H aromatics |
| 3.99 ppm, | douplet, | 4H CH₂ |
| 0.25 ppm, | triplet, | 2H OH. |

### Example 1

### Synthesis of 9,9-bis(methoxymethyl)fluorene

Into a 100 mL flask are introduced, in nitrogen atmosphere, in order: 30 mL of tetrahydrofuran (THF), 11.3 g of 9,9-bis(hydroxymethyl)fluorene, and 31.1 mL of CH₃I.

While agitating the flask content and operating at ambient temperature, one adds, in a period of 2 hours and 30 minutes, 4 g of NaH at 60% by weight in mineral oil, and the content is then allowed to react for 1 hour and 30 minutes.

The nonreacted CH₃I is recorded by distillation and the remaining content is diluted with 100 mL of water; the resulting floating solid is filtered and dried under vacuum at 40°C. After ethanol crystallization, 11.3 g of product (yield: 90%) is obtained.

The ¹H-NMR spectrum in CDCl₃, at 200 MHz and with TMS as internal standard, shows the following:

| | | |
|---|---|---|
| 7.75 ppm, | douplet, | 2H aromatics |
| 7.65 ppm, | douplet, | 2H aromatics |
| 7.39 ppm, | triplet, | 2H aromatics |
| 7.29 ppm, | triplet, | 2H aromatics |
| 3.64 ppm, | singlet, | 4H CH₂ |
| 3.35 ppm, | singlet, | 6H CH₃. |

### Comparative Example 1

### Synthesis of 9,9-bis(methoxymethyl)fluorene

Into a 250 mL flask are introduced 36 mL of a 50% aqueous solution of NaOH, 84 mL of toluene, 9.6 g of 9,9-bis(hydroxymethyl)fluorene, and 0.24 g of tetrabutylammonium hydrogen sulfate.

After heating the resulting mixture to 40°C, 8 mL of CH₃I are added dropwise over a period of 1 hour; at the end of the addition the content is allowed to react for 4 hours at 40°C.

It is then cooled to ambient temperature, diluted with 40 mL of water, and the organic phase is then separated. The aqueous phase is extracted with toluene, then the toluene extracts put together are rendered anhydrous with anhydrous Na₂SO₄ and the solvent is flashed off. After an ethanol crystallization 3 g of product (yield: 28%) is obtained.

### Comparative Example 2

### Synthesis of 9,9-bis(methoxymethyl)fluorene

Into a 250 mL flask are introduced, in anhydrous atmosphere, 100 mL of THF and 10 g of 9,9-bis(hydroxymethyl)fluorene. Then at ambient temperature 1.8 g of NaH at 60% by weight in mineral oil is added portionwise over a period of 30 minutes, and immediately thereafter 2.3 mL of CH₃I are added dropwise over a period of 30 minutes. The solution is allowed to react for 3 hours.

A second addition is then made consisting of 1.8 g of NaH and 2.3 mL of CH₃I in the same manner described above. After 3 hours of reaction time the content is diluted with 300 mL of water and then the floating solid is separated and crystallized. 5.6 g of product (yield: 50%) is obtained.

### Polymerization Example

Into a 500 mL cylindrical glass reactor equipped with a filtering barrier and a stirrer are introduced at 0°C 225 mL of TiCl₄, and, while under agitation over a period of 15 minutes, 10.1 g (54 mmoles) of microspheroidal MgCl₂·2.1 C₂H₅OH obtained as described below.

At the end of the addition, the temperature of the reactor mixture is brought to 70°C, and 9 mmoles of 9,9-bis(hydroxymethyl)fluorene are introduced. The temperature is increased to 100°C and, after 2 hours, the TiCl₄ is removed by filtration. 200 mL, of TiCl₄ and 9 mmoles of 9,9-bis(hydroxymethyl)fluorene are added again; after 1 hour at 120°C the content is filtered again and another 200 mL of TiCl₄ are added, continuing the treatment at 120°C for one more hour. Finally, the content is filtered and washed at 60°C with n-heptane until all chlorine ions disappear from the filtrate. The solid catalyst component obtained in this manner contains 3.5% by weight of Ti and 16.2% by weight of 9,9-bis(hydroxymethyl)fluorene.

The microspheroidal MgCl₂·2.1 C₂H₅OH is prepared as follows.

48 g of anhydrous MgCl₂, 77 g of anhydrous C₂H₅OH, and 830 mL of kerosene are fed, in inert gas current and at ambient temperature, into a 2 liter reactor equipped with a turbine agitator and a drawing pipe. The content is heated to 120°C while stirring thus forming the MgCl₂ alcohol adduct that melts and remains mixed with the dispersing agent. The nitrogen pressure inside the reactor is maintained at 15 atm. The drawing pipe of the reactor is heated and has an inside diameter of 1 mm, and is 3 meters long from one end of the heating jacket to the other.

Then the mixture is caused to flow through the pipe at a velocity of 7 m/sec ca.

At the exit of the pipe the dispersion is gathered in a 5 L flask, under agitation, containing 2.5 L of kerosene, and being externally cooled by way of a jacket maintained at an initial temperature of -40°C.

The final temperature of the emulsion is 0°C.

The spherical solid product that constituted the dispersed phase of the emulsion is separated by allowing it to settle and the filtrering followed by washed with heptane and drying.

All these operations are carried out in an inert gas atmosphere.

130 g of MgCl₂·3 C₂H₅OH in the form of spherical solid particles with a maximum diameter less than or equal to 50 micron is obtained.

The alcohol is removed from the product thus obtained at temperatures that are gradually increased from 50°C to 100°C in nitrogen atmosphere until the alcohol content is reduced to 2.1 moles per mole of MgCl₂.

In a 4 liter reactor, previously purged with gaseous propylene at 70°C for 1 hour, are introduced at ambient temperature and in propylene stream 70 mL of anhydrous n-hexane containing 7 mmoles of aluminum triethyl and 4 mg of the solid catalyst component prepared as described above. The reactor is closed, 1.7 NL of hydrogen and 1.2 kg of liquid propylene are introduced; the agitator is put in motion and the temperature of the reactor mixture is increased to 70°C over a period of 5 minutes. After 2 hours at 70°C, the agitation is stopped, the nonpolymerized propylene monomer is removed, and the content is cooled to ambient temperature.

380 g of polypropylene is discharged from the reactor, said polypropylene having a fraction insoluble in xylene at 25°C equal to 97.7%, and a melt index, measured according to ASTM-D 1238, condition L, of 4,5 g/10 min. The polymer yield is 95,000 g of polypropylene/g of solid catalyst component.

## Claims

1. A diether having general formula (I) 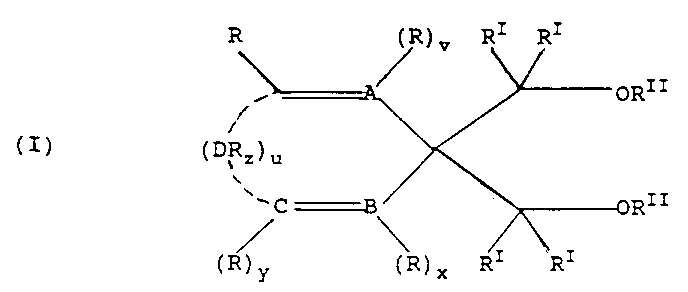 where A, B, C, and D are carbon atoms or heteroatoms selected from the group consisting of N, O, Si and S; v, x, and y are 0 or 1; u and z are either 0, 1, or 2;
provided that when u = 0:
i) A, B, and C are carbon atoms and v, x, and y are equal to 1; or
ii) A is a nitrogen atom, B and C are carbon atoms, v is equal to 0, and x and y are equal to 1; or
iii) A and B are nitrogen atoms, C is a carbon atom, v and x are equal to 0, and y is equal to 1; or
iv) A and B are carbon atoms, C is a nitrogen atom, v and x are equal to 1, and y is equal to 0;
when u = 1:
1) A, B, C, and D are carbon atoms, v, x, and y are equal to 1, and z is equal to 2; or
2) A and B are carbon atoms, C is a nitrogen atom, D is an oxygen atom, v and x are equal to 1, y and z are equal to 0; or
3) A, B, and C are carbon atoms, D is an oxygen, nitrogen, sulfur, or silicon atom, v, x, and y are equal to 1, and z is equal to 0 when D is an oxygen or sulfur atom, equal to 1 when D is a nitrogen atom, and equal to 2 when D is a silicon atom;
when u = 2:
A, B, and C are carbon atoms, D represents two carbon atoms bonded to each other by a single or double bond, v, x, and y are equal to 1, and z is 1 when the pair of carbon atoms D is bonded by a double bond, and z is 2 when said pair is bonded by a single bond;
radicals R and R^{I}, equal or different, are hydrogen; halogens; C₁-C₂₀ alkyl radicals, linear or branched; C₃-C₂₀ cycloalkyl, C₆-C₂₀ aryl, C₇-C₂₀ alkylaryl and C₇-C₂₀ aralkyl radicals; the R^{II} radicals, equal or different, are C₁-C₂₀ alkyl radicals, linear or branched; C₃-C₂₀ cycloalkyl, C₆-C₂₀ aryl; C₇-C₂₀ alkylaryl and C₇-C₂₀ aralkyl radicals, and two or more of the R radicals can be bonded to each other to form condensed cyclic structures, saturated or unsaturated, optionally substituted with R^{III} radicals wherein R^{III} is a halogen; C₁-C₂₀ alkyl radicals, linear or branched; C₃-C₂₀ cycloalkyl, C₆-C₂₀ aryl, C₇-C₂₀ alkylaryl and C₇-C₂₀ aralkyl radicals; said radicals from R to R^{III} optionally containing one or more heteroatoms as substitutes for carbon or hydrogen atoms, or both.

2. The diether of formula (I) where the heteroatoms optionally present in the radicals from R to R^{III} are selected from the group consisting of N, S, O, P, Si, Cl, or F.

3. The diether of claim 1 comprised in formula (II) 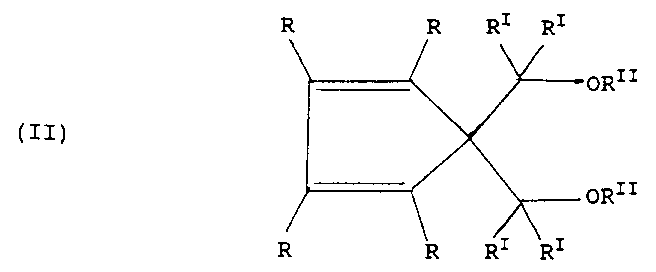 where the radicals from R to R^{II} are as defined for formula (I).

4. The diether of claim 3, where the two or more R radicals are bonded to each other to form one or more condensed cyclic structures, optionally substituted by R^{III} radicals.

5. The diether of claim 3, where the condensed cyclic structures are benzenic structures, optionally substituted by R^{III} radicals.

6. The diether of claim 4 selected from the group consisting of:
1,1-bis(methoxymethyl)-cyclopentadiene;
1,1-bis(methoxymethyl)-2,3,4,5-tetramethylcyclopentadiene;
1,1-bis(methoxymethyl)-2,3,4,5-tetraphenylcyclopentadiene;
1,1-bis(methoxymethyl)indene;
1,1-bis(methoxymethyl)-2,3-dimethylindene;
1,1-bis(methoxymethyl)-4,7-dimethylindene;
1,1-bis(methoxymethyl)-4-phenyl-2-methylindene;
1,1-bis(methoxymethyl)-7-(3,3,3-trifluoropropyl)indene;
1,1-bis(methoxymethyl)-7-trimethylsilylindene;
1,1-bis(methoxymethyl)-7-trifluoromethylindene;
1,1-bis(methoxymethyl)-7-methylindene;
1,1-bis(methoxymethyl)-7-cyclopenthylindene;
1,1-bis(methoxymethyl)-7-isopropylindene;
1,1-bis(methoxymethyl)-7-cyclohexylindene;
1,1-bis(methoxymethyl)-7-tert-butylindene;
1,1-bis(methoxymethyl)-7-tertbutyl-2-methylindene;
1,1-bis(methoxymethyl)-7-phenylindene;
1,1-bis(methoxymethyl)-2-phenylindene;
9,9-bis(methoxymethyl)fluorene;
9,9-bis(methoxymethyl)-2,3,6,7-tetramethylfluorene;
9,9-bis(methoxymethyl)-2,3,4,5,6,7-hexafluorofluorene;
9,9-bis(methoxymethyl)-2,3-benzofluorene;
9,9-bis(methoxymethyl)-2,3,6,7-dibenzofluorene;
9,9-bis(methoxymethyl)-2,7-diisopropylfluorene;
9,9-bis(methoxymethyl)-1,8-dichlorofluorene;
9,9-bis(methoxymethyl)-2,7-dicyclopentylfluorene;
9,9-bis(methoxymethyl)-1,8-difluorofluorene;
9,9-bis(methoxymethyl)-1,2,3,4-tetrahydrofluorene;
9,9-bis(methoxymethyl)-1,2,3,4,5,6,7,8-octahydrofluorene;
9,9-bis(methoxymethyl)-4-tert-butylfluorene.
1,1-bis(α-methoxybenzyl)indene;
1,1-bis(1'-methoxyethyl)-5,6-dichloroindene;
9,9-bis(α-methoxybenzyl)fluorene;
9,9-bis(1'-methoxyethyl)fluorene;
9-methoxymethyl-9-(1'-methoxyethyl)-2,3,6,7-tetrafluorofluorene;
9-methoxymethyl-9-pentoxymethylfluorene;
9-methoxymethyl-9-ethoxymethylfluorene;
9-methoxymethyl-9-(1'methoxyethyl)-fluorene; and
9-methoxymethyl-9-[2-(2-methoxypropyl)]-fluorene.

7. The diether of claim 1 selected from the group consisting of:
1,1-bis(methoxymethyl)benzonaphthene;
9,9-bis(methoxymethyl)-9,10-dihydroanthracene;
9,9-bis(methoxymethyl)xanthene; and
9,9-bis(methoxymethyl)-2,3,6,7-tetramethylxanthene.

8. A process for the synthesis of a diether of general formula (III) 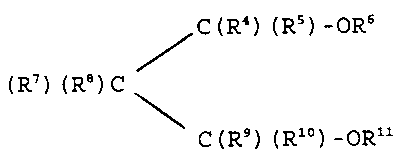 where the R⁷ and R⁸ radicals, equal or different, are hydrogen or C₁-C₂₀ alkyl radicals, linear or branched; C₃-C₂₀ cycloalkyl, C₆-C₂₀ aryl, C₇-C₂₀ alkylaryl and C₇-C₂₀ aralkyl radicals; the R⁴, R⁵, R⁹, and R¹⁰ radicals, equal or different, have the same meaning as the radicals R⁷ and R⁸ radicals; the R⁶ and R¹¹ radicals have the same meaning as defined for the R⁷ and R⁸ radicals except for the hydrogen; two or more radicals from R⁴ to R¹⁰ can be bonded to form a cyclic structure; said radicals from R⁴ to R¹¹ optionally containing one or more heteroatoms, as substitutes for one or more carbon or hydrogen atoms, or both, selected from N, O, S, P, Si and halogens;
said process comprising the following steps:
a) mixing a diol of general formula (IV) 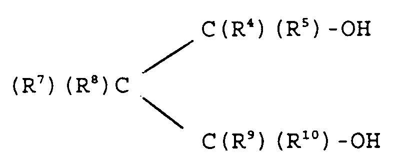 where the radicals from R⁴ to R¹⁰ are as defined for general formula (III), with a compound or a mixture of compounds selected from the compounds of general formulae R⁶X (V), or general formula R¹¹X (VI), where X is Cl, Br, I, CH₃SO₃, C₆H₅-SO₃, or p-CH₃-C₆H₄-SO₃, or of general formula (R⁶)₂SOₙ (VII), or of general formula (R¹¹)₂SOₙ (VIII), where R⁶ and R¹¹ have the meaning as defined for general formula (III), and n is 3 or 4, in a solvent which is basically nonreactive toward the reagents; and then
b) adding a base which is substantially inert towards the compounds of general formula (V) to (VIII), and is capable of forming the alcoholated derivative of the corresponding diol (IV) under the reaction conditions.

9. The process of claim 8 for the synthesis of diethers of general formula (I).

10. The process of claim 8 where the base is sodium hydride or sodium hydroxide.

11. The process of claim 8, where the solvent is selected from the group consisting of tetrahydrofuran, dimethyl sulfoxide, diethyl ether, aliphatic or aromatic hydrocarbons, and dimethylformamide.
